# FASCICULE DE BREVET EUROPEEN

(11) **EP 3 231 398 B1**
(45) Date de publication et mention de la délivrance du brevet: **26.09.2018**
(21) Numéro de dépôt: 16164981.9
(22) Date de dépôt: 12.04.2016
(51) Int. Cl.: A61F 2/46

(54) **ENSEMBLE POUR L'IMPLANTATION D'UN COTYLE DANS UNE CAVITÉ COTYLOÏDIENNE**
EINHEIT ZUR IMPLANTATION EINER GELENKPFANNE IN EINEN GELENKPFANNENHOHLRAUM
ASSEMBLY FOR IMPLANTING A COTYLE IN A COTYLOID CAVITY

(43) Date de publication de la demande: 18.10.2017
(73) Titulaire: Dedienne Sante, 34130 Mauguio (FR); AXIOM, 69004 Lyon (FR)
(72) Inventeur: BONIN, Nicolas, 69004 Lyon (FR); MANIN, Christian, 69153 Décines Cedex (FR); GAUME, Jean-Michel, 69153 Décines Cedex (FR); MONFROY, Pierre-Yves, F-69153 Decines Cedex (FR)
(74) Mandataire: Jeannet, Olivier

(56) Documents cités:
- WO-A1-2015/044685
- DE-U1-202011 050 009
- US-A1- 2013 158 558
- US-A1- 2014 364 958

## Description

La présente invention concerne un ensemble pour l'implantation d'un cotyle dans une cavité cotyloïdienne. Le terme "cotyle" utilisé dans la présente description désigne la pièce externe d'un implant cotyloïdien, généralement métallique, destinée à être impactée dans ladite cavité cotyloïdienne du bassin d'un patient. Cet implant cotyloïdien comprend, outre cette coque externe, et selon une technique bien connue qu'il n'est pas nécessaire de détailler, un noyau intérieur de glissement, notamment en polyéthylène à haute densité, formant une cavité d'articulation pour une tête fémorale portée par une tige fémorale implantée dans le fémur.

Comme on le sait, l'os du bassin d'un patient peut être amené à s'user au cours du temps au niveau de ses parties délimitant la cavité cotyloïdienne, obligeant ainsi à la mise en place d'un implant cotyloïdien venant reformer une cavité articulaire en vue de la réception de la tête d'articulation fémorale prothétique.

Pour parfaitement prendre sa place dans la cavité cotyloïdienne, l'implant cotyloïdien doit être impacté dans cette cavité, ce qui se réalise au moyen d'un instrument comprenant une tête de préhension du cotyle et un manche de manipulation/impaction de ce cotyle.

Par exemple, la publication de demande de brevet N° WO 2004/069107 décrit un cotyle à gorge interne proximale et un instrument comprenant une tête de préhension solidaire d'un manche. La tête de préhension est équipée de pattes de verrouillage du cotyle, mobiles radialement, qui viennent en engagement dans la gorge du cotyle pour assurer le montage du cotyle sur elle, et qui sont aptes à être reculées au moyen d'un manchon, pour libérer le cotyle.

Un tel instrument a une structure complexe, donc onéreuse à fabriquer. De plus, cet instrument doit être minutieusement stérilisé entre deux interventions, en particulier au niveau des nombreux interstices que comprend la tête de préhension, ce qui n'est pas sans incidence sur le coût d'utilisation de cet instrument.

La présente invention vise à remédier à cet inconvénient.

Les publications de demandes de brevet N° US 2007/219562, WO 86/05384 et US 6,468,281 décrivent des instruments similaires, à têtes de préhension solidaires de manches, ces instruments comprenant en outre des têtes d'appui distales venant au contact du fond des cotyles pour répartir l'effort d'impaction. Les mêmes commentaires que ci-dessus peuvent être formulés concernant les ensembles décrits par ces documents.

L'état de la technique le plus proche est donné par le document US 2014/0364958 A1, qui définie le préambule de la revendication 1.

La présente invention a précisément pour but de remédier aux inconvénients précités que présentent les ensembles existants.

L'ensemble concerné comprend, de manière connue en soi :
- un cotyle, destiné à être implanté dans une cavité cotyloïdienne du bassin d'un patient, et
- un instrument de préhension/impaction du cotyle, comprenant :
   - un préhenseur, présentant une surface adaptée pour venir au contact du cotyle, et équipé d'organes de retenue réversible du cotyle, et
   - un manche de préhension/impaction
- le préhenseur étant un sous-ensemble séparé du manche et assemblable à ce manche, comprenant des moyens de liaison au manche ;
- le manche étant un sous-ensemble séparé du préhenseur et assemblable au préhenseur, comprenant des moyens de liaison au préhenseur, aptes à coopérer avec les moyens de liaison que comprend le préhenseur pour relier ce manche à ce préhenseur ;
- les organes de retenue réversible étant sous la forme de dents d'encliquetage adaptées à venir en prise avec un rebord d'encliquetage que comprend le cotyle et à libérer le cotyle par suite d'une poussée exercée sur le cotyle sensiblement selon l'axe de révolution de ce cotyle.
Selon l'invention,
- le cotyle présente au moins deux rebords d'encliquetage;
   - le préhenseur comprend un poussoir mobile, déplaçable depuis une position de retrait ne faisant pas obstacle à l'assemblage du cotyle au préhenseur, vers une position d'extension, ce poussoir au cours de ce déplacement, exerçant une poussée sur le cotyle sensiblement selon l'axe de révolution de ce cotyle, de manière à désengager les rebords d'encliquetage que comprend le cotyle de leur prise avec les dents d'encliquetage que comprend le préhenseur ; et
   - l'ensemble comprend des moyens d'actionnement du poussoir mobile, permettant de déplacer ce poussoir de ladite position de retrait à ladite position d'extension.

Le préhenseur est ainsi formé par un sous-ensemble séparable du manche, de sorte qu'il est apte à être assemblé au cotyle par le fabricant du cotyle puis à être stérilisé et emballé avec ce cotyle. Lors de l'utilisation, le praticien ouvre cet emballage et amène les moyens de liaison respectifs du manche et du préhenseur en état d'assemblage mutuel, ce qui lui permet de constituer l'instrument de préhension/impaction du cotyle ; une fois l'impaction réalisée, ce praticien agit sur les moyens d'actionnement du poussoir de manière à déplacer ce poussoir vers ladite position d'extension et, ce faisant, séparer le cotyle du préhenseur.

Cette structure d'instrument permet ainsi de conférer au préhenseur une structure simplifiée, permettant notamment que ce préhenseur soit à usage unique. Il en résulte que les exigences de stérilisation de l'instrument ne concernent que le manche, et sont fortement réduites.

De plus, l'ensemble selon l'invention permet une éjection du cotyle vis-à-vis du préhenseur, donc une action de séparation de l'instrument et du cotyle ne risquant d'affecter la position du cotyle dans la cavité appareillée.

Le préhenseur peut notamment être constitué par une pièce unique réalisée par moulage ou usinage d'une matière synthétique, lesdites dents d'encliquetage formant corps avec le reste du préhenseur.

Ce préhenseur est ainsi particulièrement peu onéreux à réaliser.

Il serait concevable que lesdits moyens d'actionnement du poussoir mobile soient de type à piston ou à vissage/dévissage, par exemple agissant au travers du manche, qui serait tubulaire.

Par ailleurs, il serait concevable que lesdits moyens de liaison du manche au préhenseur soient sous la forme d'une liaison vissée ; par exemple, un manche tubulaire tel que précité serait fileté extérieurement et assemblable au préhenseur par vissage.

Toutefois, selon un mode de réalisation préférée de l'invention :
- les moyens de liaison du préhenseur au manche comprennent :
   - une cavité d'assemblage aménagée dans le préhenseur et une tête d'assemblage solidaire du manche, cette tête d'assemblage formant une extension apte à être reçue de façon ajustée dans la cavité d'assemblage ;
   - des premiers évidements de verrouillage aménagés dans les parois délimitant ladite cavité d'assemblage et des deuxièmes évidements de verrouillage aménagés dans des parois latérales de ladite extension, ces premiers et deuxièmes évidements venant en regard les uns des autres dans la position d'assemblage du préhenseur au manche ;
   - une pièce mobile de verrouillage/poussée, déplaçable dans ladite extension, qui forme des troisièmes évidements aptes à venir en regard desdits deuxièmes évidements, et qui présente des méplats situés à proximité de ces troisièmes évidements, du côté proximal par rapport à ceux-ci ;
   - un jonc déformable élastiquement, ou autre élément expansif similaire, engagé et retenu dans lesdits premiers évidements ou dans lesdits deuxièmes évidements, ce jonc étant déformable entre un état de contraction dans lequel il ne fait pas obstacle à l'engagement et au coulissement de ladite extension dans ladite cavité d'assemblage et un état normal dans lequel il s'étend à la fois dans lesdits premiers évidements et dans lesdits deuxièmes évidements, réalisant ainsi une rétention réversible de ladite extension dans ladite cavité d'assemblage et donc un assemblage réversible du préhenseur au manche ;
      ladite pièce de verrouillage/poussée étant mobile entre une position de non verrouillage de l'assemblage du préhenseur au manche, dans laquelle lesdits troisièmes évidements sont en regard du jonc et permettent la déformation de ce jonc dans ledit état de contraction, donc permettent ledit assemblage réversible du préhenseur au manche, et une position de verrouillage de l'assemblage du préhenseur au manche, dans laquelle lesdits méplats que comprend cette pièce de verrouillage/poussée sont en regard du jonc et interdisent la déformation de ce jonc dans ledit état de contraction, donc verrouillent l'assemblage du préhenseur au manche ;
- le poussoir mobile est situé en regard de ladite cavité d'assemblage ; et
- les moyens d'actionnement du poussoir incluent ladite pièce de verrouillage/poussée, qui est mobile entre ladite position de verrouillage et une position d'appui dans laquelle elle vient appuyer contre le poussoir mobile de façon à déplacer ce poussoir vers ladite position d'extension.

Ainsi, l'assemblage du manche au préhenseur est réalisé de manière simple, par engagement de ladite extension que comprend la tête d'assemblage dans ladite cavité d'assemblage, jusqu'à engagement du jonc dans lesdits premiers et deuxièmes évidements, par rappel élastique de ce jonc. Il est alors réalisé une liaison réversible du manche au préhenseur, suffisante pour saisir l'ensemble préhenseur-cotyle et amener cet ensemble en direction de la cavité cotyloïdienne à appareiller.

Une fois réalisée l'impaction du cotyle dans la cavité cotyloïdienne, le praticien agit sur les moyens d'actionnement de façon à déplacer ladite pièce de verrouillage/poussée vers ladite position de verrouillage de la liaison du préhenseur au manche, puis vers ladite position d'appui, de façon à réaliser l'éjection du cotyle. Par conséquent, la liaison du préhenseur au manche est verrouillée avant que l'effort nécessaire à l'éjection du cotyle commence à être exercé sur la liaison du cotyle au préhenseur.

L'invention fournit ainsi des moyens de liaison ayant une utilisation simple, agissant par simple inter-engagement, et dont le maintien en prise est assuré automatiquement au moment où est réalisée l'éjection du cotyle. Il n'y a donc pas de risque que ces moyens de liaison se libèrent au lieu que ce soit la liaison à encliquetage du cotyle au préhenseur qui se libère.

Il doit être souligné que c'est par souci de clarté de la description qui précède qu'il a été mentionné que ladite cavité d'assemblage est aménagée dans le préhenseur et que ladite extension est aménagée sur la tête d'assemblage ; il serait en effet concevable que ladite cavité soit aménagée dans la tête d'assemblage et que le préhenseur forme ladite extension.

De préférence, lesdits moyens d'actionnement incluent, outre ladite pièce de verrouillage/poussée, un levier monté pivotant sur le manche et une tringle reliant ce levier à la pièce de verrouillage/poussée, ladite tringle réalisant, lors du pivotement du levier, le déplacement de ladite pièce de verrouillage/poussée entre lesdites positions de non verrouillage et d'appui.

Selon une forme de réalisation simple de l'invention,
- le préhenseur comprend un alésage dans lequel le poussoir est engagé et est apte à coulisser ;
- le poussoir est formé par une pièce cylindrique présentant deux nervures circulaires faisant saillie de sa paroi, ces nervures étant situées à une distance l'une de l'autre au moins égale à la course du poussoir entre ses positions précitées de retrait et d'extension ;
- le préhenseur est en un matériau légèrement déformable élastiquement, tel qu'une matière synthétique, permettant l'engagement de l'une desdites nervures circulaires au travers dudit alésage et la rétention du poussoir sur le préhenseur par rappel élastique du matériau constituant le préhenseur autour de la portion de paroi du poussoir située entre les deux nervures.

Le préhenseur et le poussoir ont ainsi des structures simples, et le montage du poussoir sur le préhenseur est réalisé de façon simple.

Avantageusement, l'extension forme, au niveau de son extrémité destinée à être tournée vers le poussoir, un embout enveloppant propre avenir en engagement sur le poussoir.

Cette venue en engagement de l'extension avec le poussoir assure un parfait guidage du poussoir lorsque la pièce de verrouillage/poussée vient porter contre ce poussoir au cours de son déplacement.

L'invention sera bien comprise, et d'autres caractéristiques et avantages de celle-ci apparaîtront, en référence au dessin schématique annexé, décrit ci-après ; ce dessin représente, à titre d'exemple non limitatif, une forme de réalisation préférée de l'ensemble concerné.
La figure 1 est une vue de cet ensemble de côté, dans une première position d'un levier qu'il comprend ;
la figure 2 en est une vue similaire à la figure 1, dans une deuxième position du levier ;
la figure 3 est une vue en perspective, à échelle agrandie, d'une tête d'assemblage qu'il comprend ;
la figure 4 est une vue de côté, à échelle agrandie, d'un préhenseur et d'un cotyle qu'il comprend ;
la figure 5 est une vue du préhenseur en coupe selon la ligne V-V de la figure 4 ;
la figure 6 est une vue du préhenseur similaire à la figure 5, après engagement d'une extension que comprend la tête d'assemblage dans une cavité que comprend le préhenseur ;
la figure 7 est une vue de côté, en coupe passant par l'axe du cotyle, d'une tête d'assemblage que comprend le manche, du préhenseur et du cotyle, dans une position de non assemblage de la tête et du préhenseur, avec cerclage d'un détail ;
la figure 7A est une vue du détail cerclé sur la figure 7 ;
les figures 8 et 8A sont des vues similaires respectivement aux figures 7 et 7A, dans une position d'assemblage non verrouillé de ladite tête d'assemblage au préhenseur ;
les figures 9 et 9A sont des vues similaires respectivement aux figures 8 et 8A, dans une position d'assemblage verrouillé de ladite tête d'assemblage au préhenseur et d'éjection du cotyle ; et
les figures 10 et 10A sont des vues similaires respectivement aux figures 9 et 9A, dans une position de retour de ladite tête d'assemblage et du préhenseur dans une position d'assemblage non verrouillé, alors que le cotyle a été éjecté.

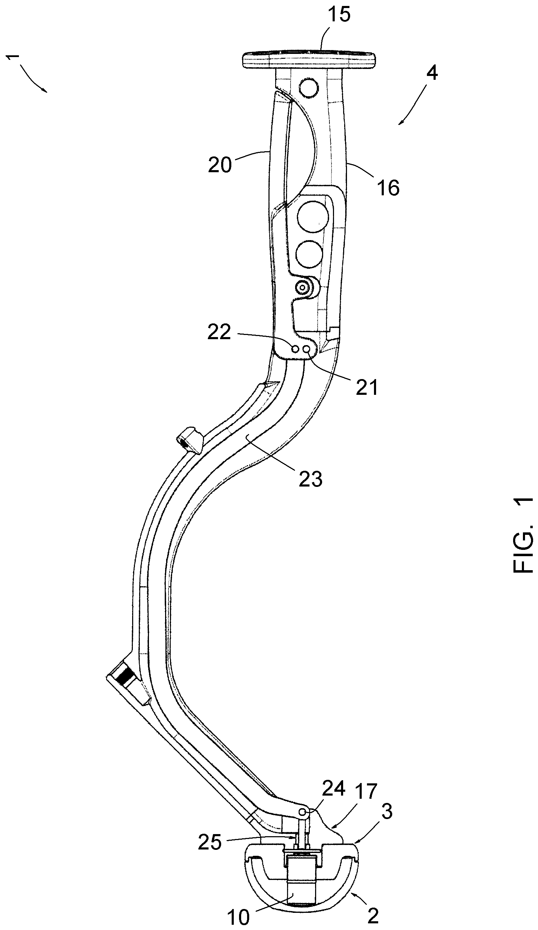

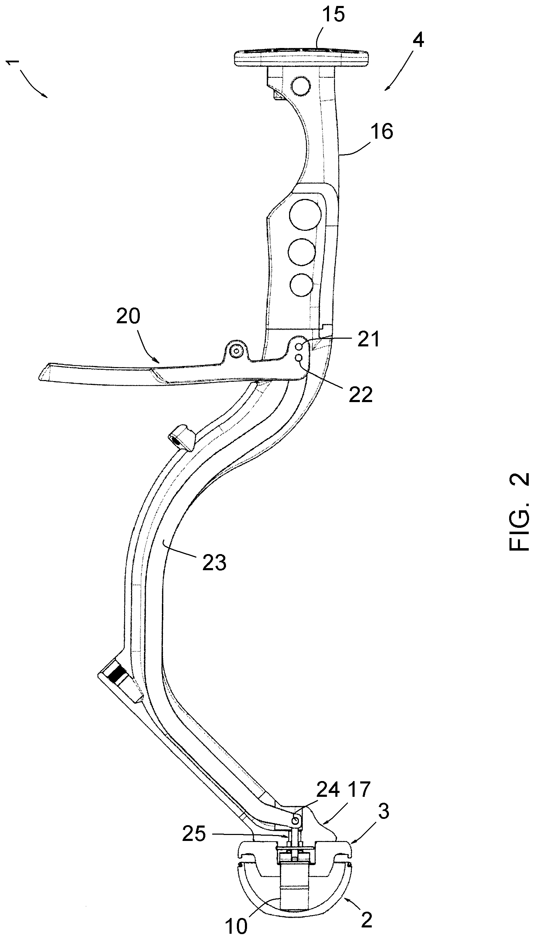

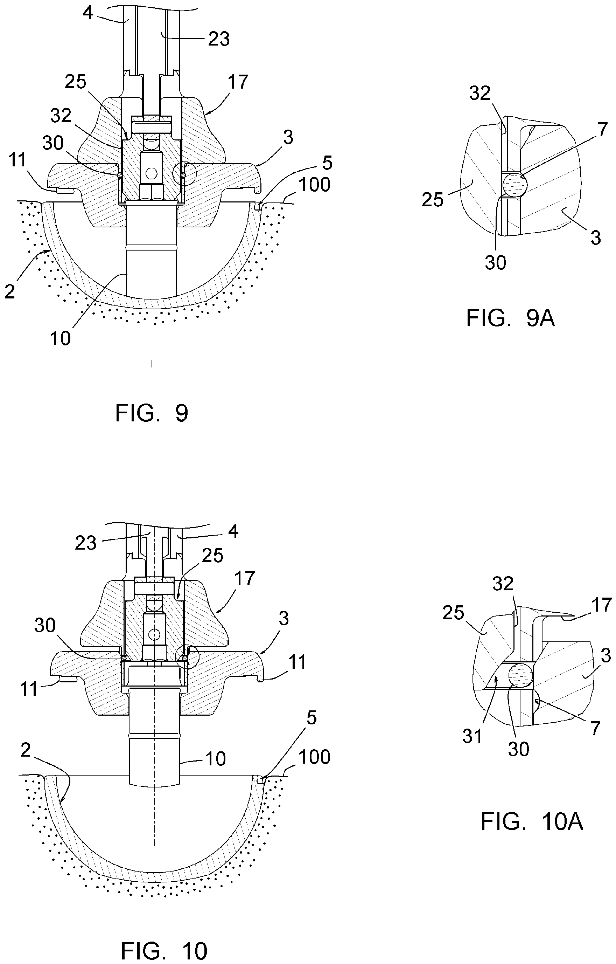

Les figures 1 et 2 représentent un ensemble 1 pour l'implantation d'un cotyle prothétique 2 dans une cavité cotyloïdienne, cette cavité étant délimitée par l'os 100 du bassin d'un patient, comme représenté schématiquement sur les figures 9 et 10.

L'ensemble 1 comprend, outre le cotyle 2, un préhenseur 3 et un manche 4.

Le cotyle 2 est formé, de manière bien connue en soi, par une coque métallique ayant, dans l'exemple représenté, une forme sensiblement hémisphérique et une paroi lisse et continue. La cavité délimitée intérieurement par ce cotyle 2 est destinée à recevoir un noyau de glissement (non représenté), notamment en polyéthylène à haute densité, formant une cavité d'articulation pour une tête fémorale portée par une tige fémorale implantée dans le fémur.

Le cotyle 2 présente, au niveau de son bord équatorial et au niveau de sa face extérieure, cinq évidements 5 régulièrement répartis sur sa circonférence, formant des nervures d'encliquetage arrondies, visibles sur les figures 9 et 10.

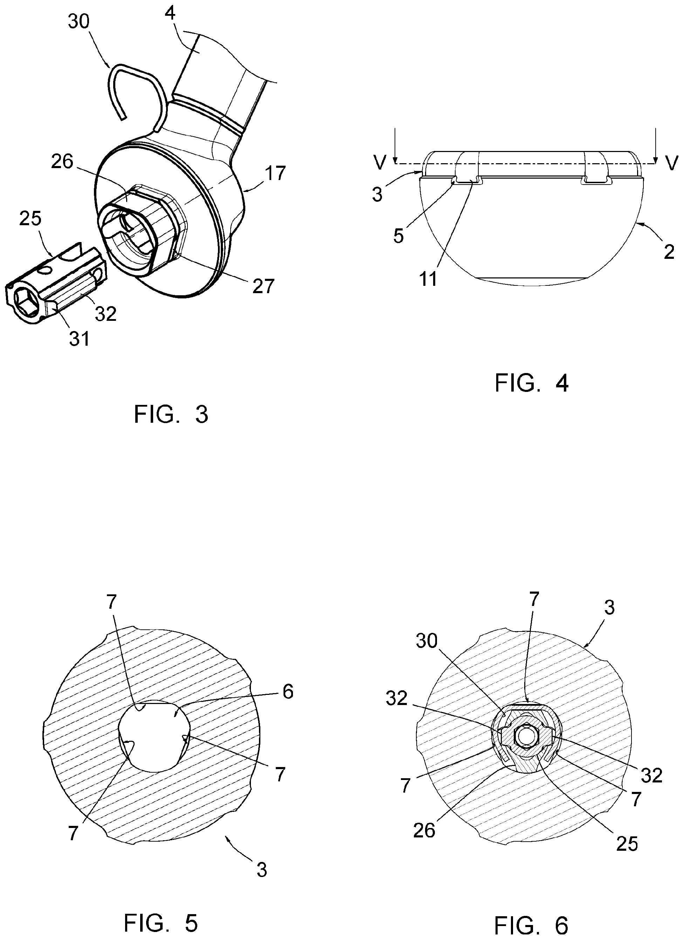

Le préhenseur 3, plus particulièrement visible sur les figures 4 et suivantes, est formé par une pièce unique de matière synthétique moulée ou usinée. Il comprend une partie proximale aplatie 3a et un bossage axial distal 3b, et forme une cavité d'assemblage 6. Au niveau des parois délimitant cette cavité 6, des premiers évidements allongés 7 sont aménagés.

La partie proximale aplatie 3a forme une surface périphérique destinée à venir en appui contre le rebord équatorial du cotyle 2, et le bossage axial distal 2b forme un alésage axial, dans lequel est engagé un poussoir 10 de façon coulissante.

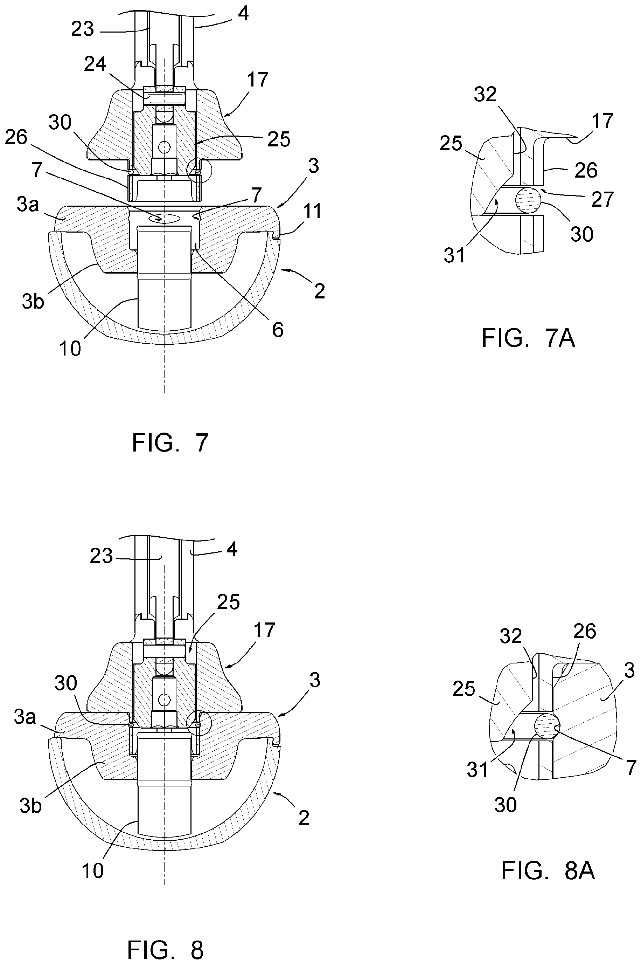

Il apparaît que ce poussoir 10 est formé par une pièce cylindrique présentant deux nervures circulaires faisant saillie de sa paroi, ces nervures étant situées à distance l'une de l'autre. Comme cela apparaît par comparaison des figures 7 et 9, cette distance est légèrement supérieure à une course que le poussoir 10 a entre une position de retrait montrée sur la figure 5 et une position d'extension montrée sur la figure 7.

Il se comprend également que le matériau constituant le poussoir 10 est légèrement déformable élastiquement, de façon à permettre l'engagement forcé de la nervure circulaire distale du poussoir au travers dudit alésage, la rétention du poussoir 10 sur le préhenseur 3 résultant du rappel élastique du matériau constituant le préhenseur 3 autour de la portion de paroi du poussoir 10 située entre les deux nervures. Pour faciliter l'engagement de ladite nervure, le préhenseur 3 forme une cuvette d'entrée au niveau de la paroi délimitant le fond de la cavité 6.

Le préhenseur 3 comprend également, au niveau du bord périphérique de sa partie proximale 3a, des dents d'encliquetage 11 destinées à venir en correspondance des évidements 5, ces dents 11 formant des rebords internes arrondis destinés à venir en prise d'encliquetage avec les rebords formés par la paroi du cotyle 2 au niveau des évidements 5, comme visible notamment sur les figures 4 et 7.

Il apparaît que, dans ladite position de retrait du poussoir 10, visible sur la figure 7, le poussoir 10 ne fait pas obstacle à l'encliquetage des dents 11 sur les rebords formés par le cotyle 2, et que l'extrémité distale du poussoir est en retrait du fond du cotyle 2

Il apparaît également que la forme arrondie des rebords respectifs des dents 11 et du cotyle 2 permet que la prise d'encliquetage soit réversible en ce sens qu'elle est libérée lorsque le poussoir 10 est déplacé vers sa position d'extension et appuie donc contre le fond du cotyle 2, comme cela se comprend par comparaison des figures 7 et 9.

En référence à nouveau aux figures 1 et 2, il apparaît que le manche 4 comprend une enclume proximale 15, une poignée de préhension 16 et une tête 17 d'assemblage au préhenseur 3.

L'enclume 15 est destinée à être frappée au moyen d'un maillet lors de l'impaction du cotyle 2 dans la cavité cotyloïdienne, alors que le manche 4 est maintenu au moyen de la poignée 16.

Comme cela est représenté, le manche 4 est équipé, au niveau de cette poignée 16, d'un levier 20 monté pivotant autour d'une broche 21, qui est relié de manière pivotante, au moyen d'une broche 22 distincte de la broche 21 et d'axe non confondu avec cette dernière, à une tringle 23. Du côté de la tête 17, cette tringle 23 est reliée à une pièce de verrouillage/poussée 25, mobile à l'intérieur de la tête 17, au moyen d'une broche 24.

Du fait du décalage des deux broches 21 et 22, il se comprend que le pivotement du levier 20 depuis la position montrée sur la figure 1 vers la position montrée sur la figure 2 provoque un mouvement de la tringle 23 en translation dans la direction distale, qui est transmis à ladite pièce de verrouillage/poussée 25 et qui réalise un déplacement de cette pièce au travers de la tête 17, comme décrit plus loin.

En référence aux figures 3, 7 et 8, il apparaît que la tête 17 présente une extension axiale 26 formant corps avec elle, apte à être engagée dans la cavité 6 de façon ajustée. Cette extension axiale 26 présente des évidements 27, dans lesquels prend place un jonc métallique 30, élastiquement déformable.

Comme visible sur la figure 3, ce jonc présente une forme en U, ayant une branche intermédiaire rectiligne et deux branches latérales courbes, et est engagé dans les évidements 27. Il apparaît sur la figure 6 que les branches latérales du jonc 30 sont conformées de façon à être aptes à venir en engagement dans les évidements 7 que forme le préhenseur 3 au niveau de ses parois délimitant cette cavité. Ce jonc 30 est déformable entre un état normal, de non déformation, visible sur les figures 3 et 6, et un état de contraction dans lequel ses branches latérales sont rapprochées l'une de l'autre, à l'intérieur des évidements 27, ceux-ci ayant une profondeur permettant ce rapprochement, comme visible sur les figures 7A ou 8A.

Il apparaît en outre que l'extension 26 forme, sur son côté distal, un embout enveloppant apte à venir en engagement sur l'extrémité proximale du poussoir 10.

La pièce de verrouillage/poussée 25 présente, comme visible sur les figures 3, 6 et 7A, deux nervures latérales situées sur deux côtés opposés, qui sont chanfreinées à leurs extrémités distales de façon à former des évidements 31 et qui présentent, du côté proximal par rapport à ces évidements 31 et immédiatement au-dessus d'eux, des faces extérieures formant des méplats 32.

Comme cela est visible sur les figures 7 et 7A, avant assemblage du manche 4 au préhenseur 3, le jonc 30 est dans son état de non déformation, dans lequel il fait partiellement saillie au-delà de la paroi de l'extension 26, voir figure 7A ; lorsque l'extension 26 est engagée dans la cavité 6, les branches latérales du jonc 30 rencontrent les parois du préhenseur 3 délimitant la cavité 6 et sont donc amenées en rapprochement l'une de l'autre en étant déplacées dans le fond des évidements 27. Lorsque la tête 17 est en position d'assemblage au préhenseur 3, comme visible sur les figures 8 et 8A, les évidements 27 viennent en regard des évidements 7 et le jonc 30 tend alors à retrouver sa forme d'origine par rappel élastique, de sorte que ses branches latérales viennent en engagement à la fois dans les évidements 27 et dans les évidements 7, voir figure 8A.

Il est alors réalisé une liaison du manche 4 au préhenseur 3, suffisante pour saisir l'ensemble préhenseur 3 - cotyle 2 et amener cet ensemble en direction de la cavité cotyloïdienne à appareiller. Cette liaison est cependant réversible du fait des formes arrondies que présentent les évidements 7 et le jonc 30 en section transversale, de sorte que la séparation de l'ensemble préhenseur 3 - cotyle 2 vis-à-vis du manche 4 reste possible, si nécessaire.

Une fois réalisée l'impaction du cotyle 2 dans la cavité cotyloïdienne, le praticien agit sur le levier 20 de façon à déplacer la pièce de verrouillage/poussée 25 dans la direction distale, ce qui, sur une première partie de la course de cette pièce 25, amène les méplats 32 en regard des branches latérales du jonc 30 et maintient ces branches latérales en engagement dans les évidements 7, comme visible sur la figure 9A ; il est ainsi réalisé un verrouillage de la liaison entre le manche 4 et le préhenseur 3. Sur la deuxième partie de la course de la pièce 25 dans la direction distale, et alors que les méplats 32 sont toujours en regard des branches latérales du jonc 30, la pièce 25 vient porter contre l'extrémité proximale du poussoir 10 et déplacer ce dernier. Au cours de ce déplacement, l'extrémité distale du poussoir 10 vient rencontrer le fond du cotyle 2 et appuyer contre ce fond de manière à libérer le cotyle 2 vis-à-vis des dents 11.

Par conséquent, la liaison du préhenseur 3 au manche 4 est verrouillée avant que l'effort nécessaire à l'éjection du cotyle 2 commence à être exercé sur la liaison du cotyle 2 au préhenseur 3.

Comme cela apparaît de ce qui précède, l'invention fournit un ensemble pour l'implantation d'un cotyle dans une cavité cotyloïdienne présentant des avantages déterminants vis-à-vis des ensembles homologues de la technique antérieure. En effet, le préhenseur 3, du fait qu'il est formé par un sous-ensemble séparable du manche 4, a une structure simplifiée, permettant que ce préhenseur soit à usage unique, ce qui réduit fortement les exigences de stérilisation, lesquelles ne concernent que le manche. De plus, l'ensemble selon l'invention permet une éjection du cotyle vis-à-vis du préhenseur, donc une action de séparation de l'instrument et du cotyle ne risquant d'affecter la position du cotyle dans la cavité appareillée.

## Revendications

1. Ensemble (1) pour l'implantation d'un cotyle (2) dans une cavité cotyloïdienne, comprenant :
- un cotyle (2), destiné à être implanté dans une cavité cotyloïdienne du bassin d'un patient, et
- un instrument de préhension/impaction du cotyle (2), comprenant :
- un préhenseur (3), présentant une surface adaptée pour venir au contact du cotyle (2), et équipé d'organes (11) de retenue réversible du cotyle (2), et
- un manche (4) de préhension/impaction ;
- le préhenseur (3) étant d'encliquetage un sous-ensemble séparé du manche (4) et assemblable à ce manche (4), comprenant des moyens (6, 7) de liaison au manche (4) ;
- le manche (4) étant un sous-ensemble séparé du préhenseur (3) et assemblable au préhenseur (3), comprenant des moyens (17, 25, 26, 27, 30, 31) de liaison au préhenseur (3), aptes à coopérer avec les moyens de liaison que comprend le préhenseur (3) pour relier ce manche (4) à ce préhenseur (3) ;
- les organes de retenue réversible étant sous la forme de dents d'encliquetage (11) adaptées à venir en prise avec un rebord d'encliquetage que comprend le cotyle (2) et à libérer le cotyle (2) par suite d'une poussée exercée sur le cotyle (2) sensiblement selon l'axe de révolution de ce cotyle (2),
**caractérisé en ce que** :
- le cotyle (2) présente au moins deux rebords d'encliquetage ;
- le préhenseur (3) comprend un poussoir mobile (10), déplaçable depuis une position de retrait ne faisant pas obstacle à l'assemblage du cotyle (2) au préhenseur (3) vers une position d'extension, ce poussoir (10), au cours de ce déplacement, exerçant une poussée sur le cotyle (2) sensiblement selon l'axe de révolution de ce cotyle (2), de manière à désengager les rebords d'encliquetage que comprend le cotyle (2) de leur prise avec les dents d'encliquetage (11) que comprend le préhenseur (3) ;et
- l'ensemble comprend des moyens (20-25) d'actionnement du poussoir mobile (10), permettant de déplacer ce poussoir de ladite position de retrait à ladite position d'extension.

2. Ensemble (1) selon la revendication 1, **caractérisé en ce que** le préhenseur (3) est constitué par une pièce unique réalisée par moulage ou usinage d'une matière synthétique, lesdites dents d'encliquetage (11) formant corps avec le reste du préhenseur (3).

3. Ensemble (1) selon la revendication 1 ou la revendication 2, **caractérisé en ce que** :
- les moyens de liaison du préhenseur (3) au manche (4) comprennent :
- une cavité d'assemblage (6) aménagée dans le préhenseur (3) et une tête d'assemblage (17) solidaire du manche (4), cette tête d'assemblage (17) formant une extension (26) apte à être reçue de façon ajustée dans la cavité d'assemblage (6) ;
- des premiers évidements de verrouillage (7) aménagés dans les parois délimitant ladite cavité d'assemblage (6) et des deuxièmes évidements de verrouillage (27) aménagés dans des parois latérales de ladite extension (26), ces premiers et deuxièmes évidements (7, 27) venant en regard les uns des autres dans la position d'assemblage du préhenseur (3) au manche (4) ;
- une pièce mobile (25) de verrouillage/poussée, déplaçable dans ladite extension (26), qui forme des troisièmes évidements (31) aptes à venir en regard desdits deuxièmes évidements (27), et qui présente des méplats (32) situés à proximité de ces troisièmes évidements (31), du côté proximal par rapport à ceux-ci ;
- un jonc (30) déformable élastiquement, ou autre élément expansif similaire, engagé et retenu dans lesdits premiers évidements (7) ou dans lesdits deuxièmes évidements (27), ce jonc (30) étant déformable entre un état de contraction dans lequel il ne fait pas obstacle à l'engagement et au coulissement de ladite extension (26) dans ladite cavité d'assemblage (6) et un état normal dans lequel il s'étend à la fois dans lesdits premiers évidements (7) et dans lesdits deuxièmes évidements (27), réalisant ainsi une rétention réversible de ladite extension (26) dans ladite cavité d'assemblage (6) et donc un assemblage réversible du préhenseur (3) au manche (4) ;
ladite pièce (25) de verrouillage/poussée étant mobile entre une position de non verrouillage de l'assemblage du préhenseur (3) au manche (4), dans laquelle lesdits troisièmes évidements (31) sont en regard du jonc (30) et permettent la déformation de ce jonc (30) dans ledit état de contraction, donc permettent ledit assemblage réversible du préhenseur (3) au manche (4), et une position de verrouillage de l'assemblage du préhenseur (3) au manche (4), dans laquelle lesdits méplats (32) que comprend cette pièce (25) de verrouillage/poussée sont en regard du jonc (30) et interdisent la déformation de ce jonc (30) dans ledit état de contraction, donc verrouillent l'assemblage du préhenseur (3) au manche (4) ;
- le poussoir (10) mobile est situé en regard de ladite cavité d'assemblage (6) ; et
- les moyens d'actionnement du poussoir (10) incluent ladite pièce (25) de verrouillage/poussée, qui est mobile entre ladite position de verrouillage et une position d'appui dans laquelle elle vient appuyer contre le poussoir (10) mobile de façon à déplacer ce poussoir (10) vers ladite position d'extension.

4. Ensemble (1) selon l'une des revendications 1 à 3 **caractérisé en ce que** ledit lesdits moyens d'actionnement incluent, outre ladite pièce (25) de verrouillage/poussée, un levier (20) monté pivotant sur le manche (4) et une tringle (23) reliant ce levier à la pièce (25) de verrouillage/poussée, ladite tringle (23) réalisant, lors du pivotement du levier (20), le déplacement de ladite pièce (25) de verrouillage/poussée entre lesdites positions de non verrouillage et d'appui.

5. Ensemble (1) selon l'une des revendications 1 à 4, **caractérisé en ce que** :
- le préhenseur (3) comprend un alésage dans lequel le poussoir (10) est engagé et est apte à coulisser ;
- le poussoir (10) est formé par une pièce cylindrique présentant deux nervures circulaires faisant saillie de sa paroi, ces nervures étant situées à une distance l'une de l'autre au moins égale à la course du poussoir (10) entre ses positions précitées de retrait et d'extension ;
- le préhenseur (3) est en un matériau légèrement déformable élastiquement, tel qu'une matière synthétique, permettant l'engagement de l'une desdites nervures circulaires au travers dudit alésage et la rétention du poussoir (10) sur le préhenseur (3) par rappel élastique du matériau constituant le préhenseur (3) autour de la portion de paroi du poussoir (10) située entre les deux nervures.

6. Ensemble (1) selon l'une des revendications 1 à 5, **caractérisé en ce que** l'extension (26) forme, au niveau de son extrémité destinée à être tournée vers le poussoir (10), un embout enveloppant propre avenir en engagement sur le poussoir (10).

## Patentansprüche

1. Anordnung (1) zur Implantation einer Gelenkpfanne (2) in einem Gelenkpfannenhohlraum, umfassend:
- eine Gelenkpfanne (2), die dazu ausgelegt ist, in einem Gelenkpfannenhohlraum des Beckens eines Patienten implantiert zu sein, und
- ein Instrument zur Ergreifung/Impaktion der Gelenkpfanne (2), umfassend:
- einen Greifer (3), der eine Oberfläche aufweist, die dazu ausgelegt ist, in Kontakt mit der Gelenkpfanne (2) zu gelangen, und mit Organen (11) zum reversiblen Halten der Gelenkpfanne (2) ausgestattet ist, und
- einen Ergreifungs-/Impaktionsschaft (4);
- wobei der Greifer (3) eine Unteranordnung ist, die vom Schaft (4) separiert und an diesem Schaft (4) anbringbar ist, umfassend Mittel (6, 7) zur Verbindung mit dem Schaft (4);
- wobei der Schaft (4) eine Unteranordnung ist, die vom Greifer (3) separiert und am Greifer (3) anbringbar ist, umfassend Mittel (17, 25, 26, 27, 30, 31) zur Verbindung mit dem Greifer (3), die dazu ausgelegt sind, mit den Verbindungsmitteln zusammenzuwirken, die der Greifer (3) umfasst, um diesen Schaft (4) mit diesem Greifer (3) zu verbinden;
- wobei die reversiblen Halteorgane die Form von Einrastzähnen (11) haben, die dazu ausgelegt sind, in Eingriff mit einem Einrastrand zu gelangen, den die Gelenkpfanne (2) umfasst, und die Gelenkpfanne (2) als Folge eines Drucks freizugeben, der auf die Gelenkpfanne (2) im Wesentlichen entlang der Drehachse dieser Gelenkpfanne (2) ausgeübt wird,
**dadurch gekennzeichnet, dass**:
- die Gelenkpfanne (2) wenigstens zwei Einrastränder aufweist;
- der Greifer (3) einen beweglichen Drücker (10) umfasst, der verlagerbar ist von einer Rückzugsposition, die die Anbringung der Gelenkpfanne (2) am Greifer (3) nicht behindert, in Richtung einer Verlängerungsposition, wobei dieser Drücker (10) im Verlauf dieser Verlagerung einen Druck auf die Gelenkpfanne (2) im Wesentlichen entlang der Drehachse dieser Gelenkpfanne (2) ausübt, derart, dass die Einrasteränder, die die Gelenkpfanne (2) umfasst, außer Eingriff mit den Einrastzähnen (11) gelangen, die der Greifer (3) umfasst; und
- die Anordnung Mittel (20-25) zur Betätigung des beweglichen Drückers (10) umfasst, die es erlauben, diesen Drücker von der Rückzugsposition zu der Verlängerungsposition zu verlagern.

2. Anordnung (1) nach Anspruch 1, **dadurch gekennzeichnet, dass** der Greifer (3) durch ein einziges Stück gebildet ist, das durch Formen oder Bearbeiten eines Kunststoffmaterials hergestellt ist, wobei die Einrastzähne (11) einen Körper mit dem Rest des Greifers (3) bilden.

3. Anordnung (1) nach Anspruch 1 oder Anspruch 2, **dadurch gekennzeichnet, dass**:
- die Mittel zur Verbindung des Greifers (3) am Schaft (4) umfassen:
- einen Montagehohlraum (6), der in dem Greifer (3) vorgesehen ist, und einen Montagekopf (17), der mit dem Schaft (4) verbunden ist, wobei dieser Montagekopf (17) eine Verlängerung (26) bildet, die dazu ausgelegt ist, in passender Weise in dem Montagehohlraum (6) aufgenommen zu sein;
- erste Verriegelungsaussparungen (7), die in den Wänden vorgesehen sind, die den Montagehohlraum (6) begrenzen, und zweite Verriegelungsaussparungen (27), die in lateralen Wänden der Verlängerung (26) vorgesehen sind, wobei diese ersten und zweiten Aussparungen (7, 27) einander in der Montageposition des Greifers (3) am Schaft (4) gegenüber liegen;
- ein bewegliches Verriegelungs-/Druckteil (25), das in der Verlängerung (26) verlagerbar ist, das dritte Aussparungen (31) bildet, die dazu ausgelegt sind, den zweiten Aussparungen (27) gegenüber zu liegen, und das Abflachungen (32) aufweist, die in der Nähe dieser dritten Aussparungen (31) an der proximalen Seite, bezogen auf selbige, angeordnet sind;
- einen elastisch verformbaren Ring (30) oder ein anderes ähnliches ausdehnungsfähiges Element, das in den ersten Aussparungen (7) oder in den zweiten Aussparungen (27) eingreift und gehalten ist, wobei dieser Ring (30) verformbar ist zwischen einem Kontraktionszustand, in dem er den Eingriff und die Verschiebung der Verlängerung (26) in den Montagehohlraum (6) nicht behindert, und einem Normalzustand, in dem er sich gleichzeitig in die ersten Aussparungen (7) und in die zweiten Aussparungen (27) erstreckt, wodurch somit ein reversibler Halt der Verlängerung (26) in dem Montagehohlraum (6) und somit eine reversible Anbringung des Greifers (3) am Schaft (4) realisiert wird;
wobei das Verriegelungs-/Druckteil (25) bewegbar ist zwischen einer Nichtverriegelungsposition der Anbringung des Greifers (3) am Schaft (4), in der die dritten Aussparungen (31) dem Ring (30) gegenüber liegen und die Verformung dieses Rings (30) in dem Kontraktionszustand ermöglichen, wodurch somit die reversible Anbringung des Greifers (3) am Schaft (4) ermöglicht wird, und einer Verriegelungsposition der Anbringung des Greifers (3) am Schaft (4), in der die Abflachungen (32), die dieses Verriegelungs-/Druckteil (25) umfasst, dem Ring (30) gegenüber liegen und die Verformung dieses Rings (30) in dem Kontraktionszustand verhindern, wodurch die Anbringung des Greifers (3) am Schaft (4) verriegelt wird;
- der bewegliche Drücker (10) gegenüber dem Montagehohlraum (6) angeordnet ist; und
- die Mittel zur Betätigung des Drückers (10) das Verriegelungs-/Druckteil (25) umfassen, das bewegbar ist zwischen der Verriegelungsposition und einer Anlageposition, in der es in Anlage gegen den beweglichen Drücker (10) derart gelangt, dass dieser Drücker (10) in Richtung der Verlängerungsposition verlagert wird.

4. Anordnung (1) nach einem der Ansprüche 1 bis 3, **dadurch gekennzeichnet, dass** die Betätigungsmittel außer dem Verriegelungs-/Druckteil (25) einen Hebel (20) umfassen, der schwenkbar an dem Schaft (4) montiert ist, sowie eine Stange (23), die diesen Hebel mit diesem Verriegelungs-/Druckteil (25) verbindet, wobei die Stange (23) während des Schwenkens des Hebels (20) die Verlagerung des Verriegelungs-/Druckteils (25) zwischen der Nichtverriegelungs- und der Anlageposition realisiert.

5. Anordnung (1) nach einem der Ansprüche 1 bis 4, **dadurch gekennzeichnet, dass**:
- der Greifer (3) eine Bohrung umfasst, in der der Drücker (10) in Eingriff und verschiebbar ist;
- der Drücker (10) durch ein zylindrisches Teil gebildet ist, das zwei kreisförmige Rippen aufweist, die von seiner Wand vorstehen, wobei diese Rippen in einem Abstand voneinander angeordnet sind, der wenigstens gleich der Wegstrecke des Drückers (10) zwischen seinen genannten Rückzugs- und Verlängerungspositionen ist;
- der Greifer (3) aus einem leicht elastisch verformbaren Material ist, wie zum Beispiel einem Kunststoffmaterial, was den Eingriff einer der kreisförmigen Rippen durch die Bohrung hindurch und das Halten des Drückers (10) am Greifer (3) durch elastische Rückstellung des Materials, das den Greifer (3) um den Bereich der Wand des Drückers (10) herum bildet, der zwischen den zwei Rippen liegt, erlaubt.

6. Anordnung (1) nach einem der Ansprüche 1 bis 5, **dadurch gekennzeichnet, dass** die Verlängerung (26) im Bereich ihres Endes, das dazu ausgelegt ist, zum Drücker (10) hin orientiert zu sein, ein einhüllendes Ansatzstück bildet, das dazu ausgelegt ist, in Eingriff am Drücker (10) zu gelangen.

## Claims

1. Assembly (1) for implanting an acetabular cup (2) in a cotyloïd cavity (6), comprising:
- an acetabular cup (2), intended to be implanted in a cotyloïd cavity (6) of a patient's pelvis, and
- a gripping/impacting instrument for the acetabular cup (2), comprising:
- a gripper (3), having an appropriate surface to come into contact with the acetabular cup (2), and equipped with reversible retaining members (11) for retaining the acetabular cup (2), and
- a gripping/impacting handle (4),
- the gripper (3) being a subassembly (1) separated from the handle (4) and capable to be assembled to said handle (4), comprising means (6, 7) for connecting to the handle (4);
- the handle (4) being a subassembly separated from the gripper (3) and capable to be assembled to the gripper (3), comprising connecting means (17, 25, 26, 27, 30, 31) for connecting to the gripper (3), capable to cooperate with the connecting means comprised by the gripper (3) to connect said handle (4) to said gripper (3);
- the reversible retaining members (11) being in the form of snapping teeth (11) suitable for engaging with a snapping rim comprised by the acetabular cup (2) and for releasing the acetabular cup (2) following a thrust exerted on the acetabular cup (2) substantially along the axis of revolution of that acetabular cup (2), **characterized in that**:
- the acetabular cup (2) has at least two snapping rims;
- the gripper (3) comprises a mobile pusher (10), movable from a withdrawn position not hindering the assembly of the acetabular cup (2) to the gripper (3), toward an extension (26) position, this pusher (10) exerting, during this movement, thrust on the acetabular cup (2) substantially along the axis of revolution of said acetabular cup (2), so as to disengage the snapping rims comprised by the acetabular cup (2) from their engagement with the snapping teeth (11) comprised by the gripper (3); and
- the assembly (1) comprises means (20-25) for actuating the moving pusher (10), making it possible to move that pusher (10) from said withdrawn position to said extension (26) position.

2. Assembly (1) according to claim 1, **characterized in that** the gripper (3) is formed by a single part made by molding or machining a synthetic material, said snapping teeth (11) being integral with the rest of the gripper (3)

3. Assembly (1) according to claim 1 or claim 2, **characterized in that**:
- the connecting means for connecting the gripper (3) to the handle (4) comprise:
- a mounting cavity (6) arranged in the gripper (3) and a mounting head (17) secured to the handle (4), this mounting head (17) forming an extension (26) capable to be received in an adjusted manner in the mounting cavity (6);
- first locking recesses (7) arranged in the walls defining said mounting cavity (6) and second locking recesses (27) arranged in the side walls of said extension (26), the first and second recesses (7, 27) coming across from one another in the assembly position of the gripper (3) to the handle (4);
- a movable locking/thrust part (25), capable to be moved in said extension (26), that forms third recesses (31) capable to come across from said second recesses (27), and that has flats (32) situated near these third recesses (31), on the proximal side relative thereto;
- an elastically deformable shank (30) or other similar expansive element, engaged and retained in said first recesses (7) or in said second recesses (27), this shank (30) being deformable between a contracted state, in which it does not hinder the engagement and sliding of said extension (26) in said mounting cavity (6), and a normal state, in which it extends both in said first recesses (7) and in said second recesses (27), thus performing a reversible retention of said extension (26) in said mounting cavity (6) and therefore a reversible assembly of the gripper (3) to the handle (4);
said locking/thrust part (25) being movable between a position not locking the assembly of the gripper (3) to the handle (4), in which said third recesses (31) are across from the shank (30) and allow the deformation of said shank (30) toward said contracted state, therefore allowing said reversible assembly of the gripper (3) to the handle (4), and a position locking the assembly of the gripper (3) to the handle (4), in which said flats (32) comprised by this locking/thrust part (25) are across from the shank (30) and prohibit the deformation of said shank (30) towards said contracted state, therefore locking the assembly of the gripper (3) to the handle (4);
- the movable pusher (10) is situated across from said mounting cavity (6); and
- the actuating means for actuating the pusher (10) include said locking/thrust part (25), which is movable between said locking position and a bearing position in which it bears against the movable pusher (10) so as to move said pusher (10) toward said extension position.

4. Assembly (1) according to one of claims 1-3, **characterized in that** said actuating means include, aside from said locking/thrust part (25), a lever (20) mounted pivoting on the handle (4) and a connecting rod (23) connecting this lever to the locking/thrust part (25), said connecting rod (23) performing, during the pivoting of the lever (20), the movement of said locking/thrust part (25) between said non-locking and bearing positions.

5. Assembly (1) according to one of claims 1-4, **characterized in that**:
- the gripper (3) comprises a bore in which the pusher (10) is engaged and is capable to slide;
- the pusher (10) is formed by a cylindrical part having two circular ribs protruding from its wall, these ribs being situated at a distance from one another at least equal to the travel of the pusher (10) between its aforementioned withdrawn and extended positions;
- the gripper (3) is made from a slightly elastically deformable material, such as a synthetic material, allowing the engagement of one of said circular ribs through said bore and the retention of the pusher (10) on the gripper (3) by elastic return of the material making up the gripper (3) around the wall portion of the pusher (10) situated between the two ribs.

6. Assembly (1) according to one of claims 1-5, **characterized in that** the extension (26) forms, at its end intended to be turned toward the pusher (10), a surrounding end-piece capable to engage on the pusher (10).
